# EUROPEAN PATENT APPLICATION

(11) **EP 2 095 808 A1**
(43) Date of publication of application: **02.09.2009**
(21) Application number: 08300116.4
(22) Date of filing: 26.02.2008
(51) Int. Cl.: A61K 8/35, A61K 8/92, A61Q 19/04

(54) **Self tanning compositions containing oils**

(71) Applicant: Johnson & Johnson Consumer France SAS, 92787 Issy les Moulineaux Cedex 9 (FR)
(72) Inventor: Magnant, Stéphanie, 27400, LA VACHERIE (FR); Quirin, Nathalie, 27400, HONDOUVILLE (FR); Perrodin, Françoise, 78930, BREUIL BOIS ROBERT (FR)
(74) Representative: Ahner, Francis

(57) **Abstract**

The invention concerns a sunless skin tanning oil-in-water emulsion comprising DHA, and a vegetable oil comprising a C₁₈ unsaturated fatty acid fraction, wherein the C₁₈ unsaturated fatty acid fraction is present in a weight ratio of C₁₈ unsaturated fatty acid fraction to DHA that is at least about 1:2.

## Description

Because of the risks associated with sun tanning such as sunburn, many people use self-tanning compositions as a means to either achieve a tan without exposure to the sun, obtain a deeper tan with less exposure to the sun, or to extend the natural life of their suntan.

Products that are currently used for sunless tanning of the skin are based on the reaction of an active chemical present in the product with the skin's amino acids. Such chemicals are well known and include compounds having an aldehyde group, or compounds having a ketone group, such as, for example, dihydroxyacetone (DHA). When applied, DHA interacts with dead skin cells located in the stratum corneum of the epidermis. This interaction causes a color change thereby providing a darkening or tanning effect which typically occurs about 2 to 4 hours after application. The color change typically lasts from about five to seven days from the initial application

However tanning agents such as DHA possess the following drawbacks:
- staining of the clothes;
- non homogenous tanning of the skin;
- unpleasant smell.
Therefore, there is a need of sunless tanning composition which contains smaller quantity of tanning agents, while providing the same color intensity after its application on the skin.

Surprisingly, the inventors have found that by using a vegetable oil comprising a C₁₈ unsaturated fatty acid fraction in a composition containing tanning agents, it is possible to use DHA as the only tanning agent and to decrease significantly the quantity of DHA in such a composition, while keeping the same color intensity when such a composition is applied on the skin.

Therefore the present invention concerns a sunless skin tanning oil-in-water emulsion comprising: a) DHA, and b) a vegetable oil comprising a C₁₈ unsaturated fatty acid fraction, wherein the C₁₈ unsaturated fatty acid fraction is present in a weight ratio of C₁₈ unsaturated fatty acid fraction to DHA that is at least about 1:2.

DHA (dihydroxyacetone) is a colorless sugar (triose carbohydrate) having the chemical formula C₃H₆O₃. It can be prepared by the mild oxidation of glycerol, for example with hydrogen peroxide and a ferrous salt as a catalyst. The chemical formula for DHA is illustrated below:

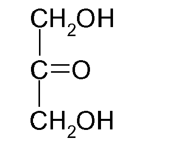

Advantageously, the oil-in-water emulsion according to the present invention comprises from 0.5 to 5 % by weight of DHA relative to the total weight of the emulsion, in particular from 0.5 to 4 % by weight of DHA relative to the total weight of the emulsion. More advantageously, the oil-in-water emulsion comprises less than 2 % by weight of DHA relative to the total weight of the emulsion, still more advantageously less than 1.6 % by weight of DHA relative to the total weight of the emulsion, in particular less than 1.5 % by weight of DHA relative to the total weight of the emulsion.

In an advantageous embodiment, the vegetable oil according to the present invention contains more than 40 % of monounsaturated and diunsaturated C18 fatty acids.

Advantageously, the vegetable oil is chosen such that the C₁₈ unsaturated fatty acid fraction therein is present in a weight ratio of C₁₈ unsaturated fatty acid fraction to DHA that is at least about 1:2, preferably at least about 1:1.5; more preferably at least about 1:1.

For example, the vegetable oil may be selected from the group consisting of avocado oil, borage oil, cottonseed oil, sesame oil and a mixture thereof, more advantageously in the group consisting of avocado oil, borage oil and a mixture thereof, still more advantageously it is a mixture of two or more vegetable oils, advantageously a mixture of avocado oil and borage oil.

In particular, borage oil is borage seed oil (Borago Officinalis seed oil) and is sold under the trade name Akostar P and avocado oil (Persea Gratissima) is sold under the trade name refined avocado oil by SICTIA.

The following Table 1 indicates the unsaturated fatty acid content (in weight %) of avocado oil and of borage oil:

**Table 1**

| | Fatty acids weight % | | | | | | | | |
|---|---|---|---|---|---|---|---|---|---|
| **oils** | Saturated C16 | Monounsaturated C16 | Saturated C18 | Monounsatu rated C18 | Diunsaturated C18 | Triunsaturated C18 | Monounsaturated C20 | Saturated C22 | Mono unsaturated C22 |
| **Acid** | Palmitic acid | Palmitoleic acid | Stearic acid | Oleic acid | Linoleic acid | Linolenic acid | eicosanoic acid | behenic acid | |
| **Avocado oil *Persea gratissima*** | 7-32 | 2-13 | <1.5 | 36-80 | 6-18 | 0-5 | - | <0.7 | |
| **Borage oil *Borago officinalis*** | 9-15 | <0.4 | 3-7 | 15-19 | 32-38 | 18-25 | 2-4 | 2-4 | |

In one embodiment, the oil-in-water emulsion according to the present invention comprises from 1 to 10 % by weight of vegetable oil relative to the total weight of the emulsion, advantageously from 2 to 8 % by weight of the vegetable oil relative to the total weight of the emulsion, still more advantageously from 4 to 7% by weight of the vegetable oil relative to the total weight of the emulsion, in particular from 5 to 7% by weight of the vegetable oil relative to the total weight of the emulsion.

If the quantity of vegetable oil in the oil-in-water emulsion according to the present invention is higher than 10% by weight, the oil-in-water emulsion according to the present invention may present a strong smell, which may not be acceptable by the consumer and therefore may need to be masked by another component of the oil-in-water emulsion according to the present invention.

In case the vegetable oil according to the present invention is a mixture of vegetable oils, the ratio of each oil in the oil mixture is advantageously of between 1/4 to 3/4, still more advantageously of between 1/3 to 2/3.

Advantageously, the vegetable oil comprises a linoleic acid fraction, wherein the linoleic acid fraction is present in a weight ratio of linoleic acid fraction to DHA that is at least about 1:3, preferably at least about 1:2, more preferably at least about 1:1.5.

In one embodiment, the oil-in-water emulsion according to the present invention is free of color additive.

The term "free of color additive" is intended to mean, in the sense of the present invention that the oil-in-water emulsion according to the present invention does not contain any color additive which: 1) can provide the skin with an immediate tanning appearance before the DHA tanning takes effect, or 2) provides the emulsion with a particular color (e.g., white, off-white, light brown, dark brown, tan, creme, caramel, yellow, orange, blue, green, red, etc.). Therefore in one embodiment, the emulsion according to the present invention is transparent or white. The color additives which are excluded from the emulsion according to the present invention are organic additive, inorganic additive and mica. Non-limiting examples of color additives according to the present invention are described in the International Cosmetic Ingredient Dictionary and Handbook, 10^{th} Edition (2004), which is incorporated into this document by reference. Examples of organic color additives include water soluble dyes, oil soluble dyes, and pigments. Inorganic color additives can produce "earthy" tones which can be used to achieve muted colors. For instance, iron oxides (e.g., red, black, and yellow oxides) in combination with titanium dioxide can be used to achieve a brownish tint. Non-limiting examples of other inorganic color additives include pigments such manganese, ultramarines, titanium dioxides, ferric ferrocyanide, and chromium oxide. Micas use light reflection, refraction, and transmission to exhibit their effects.

In another advantageous embodiment, the oil-in-water emulsion according to the present invention is free of mineral oil and petrolatum.

By formulating the present emulsion as free of mineral oil and petrolatum, the emulsions are non-irritating to sensitive skin, have an enhanced skin-feel when applied, and/or are less occlusive to DHA.

In a particular embodiment, the oil-in-water emulsion according to the present invention comprises a gelling agent chosen in the group consisting of a modified starch gelling agent, a branched polysaccharide gelling agent and a mixture thereof. Advantageously, the oil-in-water emulsion according to the present invention comprises a mixture of a modified starch gelling agent and a branched polysaccharide gelling agent.

The modified starch gelling agent used in the emulsion according to the present invention can be for example a crosslinked starch, modified corn starch, hydroxypropylated starch, or a starch phosphate ester. In one embodiment, the modified starch gelling agent is a nonionic polymer. In another embodiment, the modified starch gelling agent has shear thinning properties. In a further embodiment, the modified starch gelling agent is both nonionic and shear thinning. Preferably, the modified starch gelling agent is hydroxypropyl starch phosphate. Hydroxypropyl starch phosphate is commercially available under the name STRUCTURE XL from National Starch and Chemical.

Advantageously, the amount of modified starch gelling agent in the emulsion of the invention varies from 0.1 to 6, more advantageously from 0.5 to 3, still more advantageously of from 1 to 3, weight percent based on the total weight of the emulsion.

According to another embodiment of the invention the emulsion may contain in addition to or in place of the modified starch gelling agent, a branched polysaccharide gelling agent. For example, the branched polysaccharide gelling agent may be selected from xanthan gum and succinoglycan. Preferably, the branched polysaccharide gelling agent is succinoglycan. Succinoglycan is commercially available under the name RHEOZAN SH from Rhodia. The amount of branched polysaccharide gelling agent in the emulsion may vary from 0.01 to 1, preferably from 0.12 to 0.20, weight percent based on the total weight of the emulsion.

The emulsion of the invention may contain additional gelling agents, e.g., based on polyacrylates.

The emulsion of the invention may contain various other optional ingredients known in the art, such as acidifying agents, alkalizing agents, antimicrobial agents, antioxidants, buffering agents, chelating agents, dermotologically active agents, dispersing agents, emollients, emulsifying agents, humectants, fragrances, preservatives, sugars, sunscreen agents, surfactants, suspending agents, thickening agents, and vehicles. Examples of these ingredients are listed below as well as in the ICT Handbook.

Acidifying and alkalizing agents can be added to obtain the desired pH of the emulsion. Examples of acidifying agents included citric acid, lactic acid, glycolic acid, acetic acid, glacial acetic acid, malic acid, and propionic acid. Examples of alkalizing agent include edetol, potassium carbonate, potassium hydroxide, sodium borate, sodium carbonate, sodium citrate, sodium lactate, sodium glycolate, and sodium hydroxide. Other acidifying and alkalizing agents are listed on page 1653 of the ICT handbook.

Antimicrobial agents can be used when the emulsion is to be applied to skin prone to microbial infection, e.g., by bacteria, fungal, or protozoa. Examples of such agents include benzyl alcohol, chlorobutanol, phenylethyl alcohol, phenylmercuric acetate, potassium sorbate, and sorbic acid, benzoic acid, butylparaben, ethylparaben, methylparaben, propylparaben, and sodium benzoate. Other antimicrobial agents are listed on page 1612 of the ICT handbook.

Antioxidants can be used to protect ingredients of the emulsion from oxidizing agents that are included within or come in contact with the emulsion. Examples of antioxidants include water soluble antioxidants such as ascorbic acid, sodium sulfite, metabisulfite, sodium miosulfite, sodium formaldehyde, sulfoxylate, isoascorbic acid, isoascorbic acid, cysteine hydrochloride, 1,4-diazobicyclo-(2,2,2)-octane, and mixtures thereof. Examples of oil-soluble antioxidants include ascorbyl palmitate, butytlated hydroxyanisole, butylated hydroxytoluene, potassium propyl gallate, octyl gallate, dodecyl gallate, phenyl-α-napthyl-amine, and tocopherols such as α-tocopherol. Other antioxidants are listed on pages 1612-13 of the ICT Handbook.

Buffering agents can be used to maintain an established pH of the emulsion. Examples of buffering agents included calcium acetate, potassium metaphosphate, potassium phosphate monobasic, and tartaric acid. Other buffering agents are listed on page 1612 of the ICT handbook.

Chelating agents can be used to maintain the ionic strength of the emulsion and/or bind to destructive compounds and metals that are included within or come in contact with the emulsion. Examples of chelating agents included edetate dipotassium, edetate disodium, edetic acid, and ethylenediamine tetraacetic acid (EDTA) and its salts (e.g., tetrasodium EDTA). Other chelating agents are listed on page 1626 of the ICT handbook.

Dermotalogically active agents include agents for treating wound healing, inflammation, acne, psoriasis, cutaneous aging, skin cancer, impetigo, herpes, chickenpox, dermatitis, pain, itching, skin irritation. Examples of such dermatalogically active agents include hydrocortisone, dexamethesone, panthenol, phenol, tetracycline hydrochloride, yeast, hexylresorcinol, lamin, kinetin, betamethasone, triamcinolone, fluocinolone, methylprednisolone, retinoids such as retinol and retinoic acid, dapsone, sulfasalazine, resorcinol, salicylic acid, benzoyl peroxide, erythromycin-benzoyl peroxide, erythromycin, clindamycin, mupirocin, griseofulvin, azoles such as miconazole, econozole, itraconazole, fluconazole, and ketoconazole, ciclopirox, allylamines such as naftifine and terfinafine, acyclovir, famciclovir, valacyclovir, benzocaine, lidocaine, dibucaine, pramoxine hydrochloride, methyl salicylate, camphor, menthol, resocinol, and vitamins such as tocopherol, and tocopherol acetate.

Examples of dispersing and suspending agents include poligeenan, magnesium aluminum silicate xanthum gum, and silicon dioxide. Other dispersing or suspending agents are listed on page 1612 of the ICT handbook.

Emollients are agents that soften and smooth the skin. Examples of emollients include oils and waxes such as microcrystaline wax, polyethylene, triglyceride esters such as those of castor oil, cocoa butter, safflower oil, corn oil, olive oil, cod liver oil, almond oil, palm oil, squalene, and soybean oil, acetylated monoglycerides, ethoxylated glycerides, fatty acids, alkyl esters of fatty acids, alkenyl esters of fatty acids, fatty alcohols, fatty alcohol ethers, ether-esters, lanolin and derivatives of lanolin, polyhydric alcohol esters, wax esters such as beeswax, vegetable waxes, phospholids, and sterols, isopropyl palmitate or glyceryl stearate. Other emollients are listed on pages 1656-61 of the ICT handbook.

Emulsifying agents can be used for preparing the oil-in-water emulsions of the present invention. Examples of emulsifying agents include Arlacel 165 and methyl gluceth sesquisterate, fatty alcohols, fatty alcohols and alkyl phenols condensed with ethylene oxide. Other emulsifiers are listed on pages 1679-87 of the ICT Handbook. Emulsion stabilizers are listed on pages 1634-35 of the ICT Handbook.

Humectants are agents that typically promote the retention of moisture, e.g., moisturizers. Examples of humectants include sorbitol, glycerin, glycereth 5 lactate, glycereth 7 triacetate, glycereth 7 diisononoate, hexanetriol, glycols such as methyl- propanediol, 1,2-pentanediol, hexylene glycol, and propylene glycol, alkoxylated glucose, D-panthenol and derivatives thereof, and hyaluronic acid.

Other humectants are listed on pages 1661-62 of the ICT Handbook.

Siloxane are particularly preferred humectant. Siloxanes that may be used in the present invention include, but are not limited to, dimethicone, cyclomethicone, phenyl trimethicone, phenyl dimethicone, cetyl dimethicone, stearyl dimethicone, amodimethicone, C₃₀₋₄₅ alkyl dimethicone, C₃₀₋₄₅ Alkyl Methicone, Cetearyl methicone, dimethicone copolyol, cyclopentasiloxane, cyclohexasiloxane or any combinations thereof.

Examples of fragrances or perfume include peppermint, rose oil, rose water, aloe vera, clove oil, menthol, camphor, eucalyptus oil, and other plant extracts. To eliminate certain odors from emulsions, masking agents may be used. An example of a masking agent includes ethylene brassylate. Other fragrances and masking agents are listed on pages 1639-40 of the ICT Handbook.

Preservatives can be used to protect the emulsion from degradation. Examples of preservatives include phenoxyethanol, methylparaben, benzalkonium chloride, benzethonium chloride, propyl paraben, benzoic acid, benzyl alcohol, and mixtures thereof such as liquipar oil. Other preservatives are listed on pages 1654-55 of the ICT Handbook.

Sugars can be used to improve the results obtained by DHA. Examples of sugars include monosaccharides, disaccharides, and polysaccharides such as glucose, xylose, fructose, reose, ribose, pentose, arabinose, allose, tallose, altrose, mannose, galactose, lactose, sucrose, erythrose, glyceraldehyde, or any combination thereof. However, advantageously the emulsion according to the present invention is free of sugars.

Sunscreen agents are agents typically used to block or reduce the amount of ultraviolet radiation impinging on the skin (e.g., by absorption, scattering, and reflection of the ultraviolet radiation). Segarin, et al., Cosmetics Science and Technology, Chapter VIII, pages 189, et seq. discloses numerous examples of sunscreen agents. Examples of sunscreen agents include both organic compounds and their salts such as, butylmethoxydibenzoyl methane, diethyl hexyl butamido triazone, diethyl amino hydroxybenzoyl hexyl benzoate, ethylhexyl triazone, bis-ethylhexyloxyphenol methoxyphenyl triazine, methylene bis-benzotriazolyl tetramethylbutyl phenol, phenyl benzymidazole sulfonic acid, ethylhexyl salicylate, benzophenone-3, homosalate, octocrylene, avobenzone, and menthyl anthranilate, as well as inorganic particulate materials such as zinc oxide, silica, iron oxide, titanium dioxide, and 2-ethyl-hexyl-p-methoxycinnamate. Other agents are listed on page 1672 of the ICT Handbook.

Generally, the emulsion may contain from 1% to 50%, by weight, of sunscreen agent(s). The exact amounts will vary depending on the sunscreen used and the desired sun-protection factor (SPF).

Surfactants are agents typically used to stabilize emulsion, e.g., used as wetting agents, antifoam agents, emulsifiers, dispersing agents, and penetrates. Examples of surfactants include lapyrium chloride, laureth 4, laureth 9, monoethanolamine, nonoxynol 4, nonoxynol 9, nonoxynol 10, nonoxynol 15, nonoxynol 30, poloxalene, polyoxyl 8, 40, and 50 stearate, polysorbate 20, polysorbate 40, polysorbate 60, polysorbate 65, polysorbate 80, and polysorbate 85, sodium lauryl sulfate, sorbitan, decyl polyglucoside, PPG-5-ceteth-20 and its derivatives. Other surfactants are listed on pages 1672-90 of the ICT Handbook.

Vehicles are often referred to as the base for the cosmetically acceptable carrier, e.g., a fluid that is capable of delivering the other components of the emulsion to the skin with acceptable absorption of those components into the skin. Examples of vehicles include water, e.g., deionized water or demineralized water. In the oil-in-water emulsion according to the present invention, the continuous water phase contains the water soluble agents and the discontinuous oil phase contains the oil soluble agents.

The viscosity of the emulsion may be different dependent on the type of formulation being prepared, e.g., a liquid formulation will have a lower viscosity than a gel formulation. Typically, the viscosity of cream formulations ranges from 5,000 to 150,000 cps. Lower viscosities, such as down to about 500 cps are also suitable for sprays, foams, mists and the like. Bulking agents may be used to increase the viscosity of the emulsion. An example of a bulking agent is talc. Other bulking agents are listed on page 1625-26 of the ICT Handbook. Other viscosity increasing agents are listed on pages 1693-97 of the ICT Handbook. Viscosity decreasing agents are listed on pages 1692-92 of the ICT Handbook.

Advantageously the oil-in-water emulsion according to the present invention is included in a cosmetic composition or product.

The present invention concerns also a method of tanning skin comprising applying the oil-in-water emulsion according to the present invention onto the skin.

Finally, the present invention concerns the use of a vegetable oil comprising a C₁₈ unsaturated fatty acid fraction to intensify the coloring action of DHA on the skin, wherein the C₁₈ unsaturated fatty acid fraction is present in a weight ratio of C₁₈ unsaturated fatty acid fraction to DHA that is at least about 1:2.. In fact by the use of the vegetable oil according to the present invention, in particular in an oil-in-water emulsion which contains DHA, it is possible to increase the color intensity of the tanning action of DHA, when a composition and in particular an oil-in-water emulsion containing DHA, is applied on the skin. Therefore, there is a synergic action between the vegetable oil according to the present invention and DHA.

The following non-limiting examples further illustrate the invention.

### Example 1: Emulsions according to the present invention

Tables 2 to 6 illustrate emulsions according to the present invention (ingredients and weight %).

**Table 2**

| **INCI Name** | **%wt** |
|---|---|
| Water | 40.0000 |
| Phenoxyethanol | 0.5000 |
| Water; Hydroxypropyl Starch Phosphate | 2.5000 |
| Glycerin | 6.0000 |
| Succinoglycan | 0.1500 |
| Sodium Hydroxide | 0.0070 |
| Water | 0.0163 |
| p-Anisic Acid | 0.1000 |
| Glyceryl Caprylate | 1.0000 |
| Sorbitol; Water | 1.5000 |
| PEG-100 Stearate; Glyceryl Stearate | 1.7000 |
| Arachidyl Alcohol; Behenyl Alcohol; Arachidyl Glucoside | 3.0000 |
| Stearyl Alcohol | 1.5000 |
| Isopropyl Palmitate | 2.0000 |
| Ethylhexyl Stearate | 3.0000 |
| Cyclopentasiloxane | 4.0000 |
| Ascorbyl Palmitate; Lecithin; Tocopherol | 0.0200 |
| Persea Gratissima (Avocado) Oil | 4.0000 |
| Borago Officinalis (Borage) Seed Oil; Mixed Tocopherols | 2.0000 |
| Water | 25.8067 |
| Dihydroxyacetone | 0.8000 |
| Perfume | 0.4000 |
| Total | 100.0000 |

**Table 3**

| **INCI Name** | **%wt** |
|---|---|
| Water | 40.0000 |
| Phenoxyethanol | 0.5000 |
| Water; Hydroxypropyl Starch Phosphate | 2.5000 |
| Glycerin | 6.0000 |
| Succinoglycan | 0.1500 |
| Sodium Hydroxide | 0.0070 |
| Water | 0.0163 |
| p-AnisicAcid | 0.1000 |
| Glyceryl Caprylate | 1.0000 |
| Sorbitol; Water | 1.5000 |
| PEG-100 Stearate; Glyceryl Stearate | 1.7000 |
| Arachidyl Alcohol; Behenyl Alcohol; Arachidyl Glucoside | 3.0000 |
| Stearyl Alcohol | 1.5000 |
| Isopropyl Palmitate | 2.0000 |
| Ethylhexyl Stearate | 3.0000 |
| Cyclohexasiloxane; Cyclopentasiloxane | 2.0000 |
| Cyclopentasiloxane; Dimethicone Crosspolymer | 2.0000 |
| Ascorbyl Palmitate; Lecithin; Tocopherol | 0.0200 |
| Persea Gratissima (Avocado) Oil | 4.0000 |
| Borago Officinalis (Borage) Seed Oil; Mixed Tocopherols | 2.0000 |
| Water | 25.4067 |
| Dihydroxyacetone | 1.2000 |
| Perfume | 0.4000 |
| Total | 100.0000 |

**Table 4**

| **INCI Name** | **%wt** |
|---|---|
| Water | 40.0000 |
| Phenoxyethanol | 0.7000 |
| Water; Hydroxypropyl Starch Phosphate | 2.5000 |
| Glycerin | 6.0000 |
| Succinoglycan | 0.1500 |
| Sodium Hydroxide | 0.0070 |
| Water | 0.0163 |
| Propylparaben | 0.1000 |
| Methylparaben | 0.4000 |
| Sorbitol; Water | 1.5000 |
| PEG-100 Stearate; Glyceryl Stearate | 1.7000 |
| Arachidyl Alcohol; Behenyl Alcohol; Arachidyl Glucoside | 3,0000 |
| Cetyl Alcohol; Stearyl Alcohol; Myristyl Alcohol | 1.5000 |
| Isopropyl Palmitate | 2.0000 |
| Ethylhexyl Stearate | 3.0000 |
| Cyclopentasiloxane | 2.0000 |
| Cyclopentasiloxane, Dimethicone Crosspolymer | 2.0000 |
| Ascorbyl Palmitate; Lecithin; Tocopherol | 0.0200 |
| Persea Gratissima (Avocado) Oil | 4.0000 |
| Borago Officinalis (Borage) Seed Oil; Mixed Tocopherols | 2.0000 |
| Water | 25.5067 |
| Dihydroxyacetone | 1.5000 |
| Perfume | 0.4000 |
| Total | 100.0000 |

**Table 5**

| **INCI Name** | **%wt** |
|---|---|
| Water | 40.0000 |
| Phenoxyethanol | 0.7000 |
| Water; Hydroxypropyl Starch Phosphate | 2.5000 |
| Glycerin | 6.0000 |
| Succinoglycan | 0.1500 |
| Sodium Hydroxide | 0.0070 |
| Water | 0.0163 |
| Propylparaben | 0.1000 |
| Methylparaben | 0.4000 |
| Sorbitol; Water | 1.5000 |
| PEG-100 Stearate; Glyceryl Stearate | 1.7000 |
| Arachidyl Alcohol; Behenyl Alcohol; Arachidyl Glucoside | 3.0000 |
| Cetyl Alcohol; Stearyl Alcohol; Myristyl Alcohol | 1.5000 |
| Isopropyl Palmitate | 2.0000 |
| Ethylhexyl Stearate | 3.0000 |
| Cyclopentasiloxane; Dimethicone Crosspolymer | 4.0000 |
| Ascorbyl Palmitate; Lecithin; Tocopherol | 0.0200 |
| Persea Gratissima (Avocado) Oil | 4.0000 |
| Borago Officinalis (Borage) Seed Oil; Mixed Tocopherols | 2.0000 |
| Water | 25.8067 |
| Dihydroxyacetone | 1.2000 |
| Perfume | 0.4000 |
| Total | 100.0000 |

**Table 6**

| **INCI Name** | **%wt** |
|---|---|
| Water | 40.0000 |
| Phenoxyethanol | 0.7000 |
| Water; Hydroxypropyl Starch Phosphate | 2.5000 |
| Glycerin | 6.0000 |
| Succinoglycan | 0.1500 |
| Sodium Hydroxide | 0.0070 |
| Water | 0.0163 |
| Propylparaben | 0.1000 |
| Methylparaben | 0.4000 |
| Sorbitol; Water | 1.5000 |
| PEG-100 Stearate; Glyceryl Stearate | 1.7000 |
| Arachidyl Alcohol; Behenyl Alcohol; Arachidyl Glucoside | 3.0000 |
| Stearyl Alcohol | 1.5000 |
| Isopropyl Palmitate | 2.0000 |
| Ethylhexyl Stearate | 3.0000 |
| Cyclohexasiloxane; Cyclopentasiloxane | 4.0000 |
| Ascorbyl Palmitate; Lecithin; Tocopherol | 0.0200 |
| Persea Gratissima (Avocado) Oil | 4.0000 |
| Borago Officinalis (Borage) Seed Oil; Mixed Tocopherols | 2.0000 |
| Water | 24.0067 |
| Dihydroxyacetone | 3.0000 |
| Perfume | 0.4000 |
| Total | 100.0000 |

### Example 2 Skin Darkening

Emulsions according to the present invention and comparative examples of emulsions not containing vegetable oil were prepared and testing for skin darkening activity. The following Tables 7-11 show the ingredients for each of the emulsions. The emulsions of Tables 7, 9 and 10 were according to the invention. The emulsions of Tables 8 and 11 were comparative.

**Table 7: an emulsion according to the present invention with avocado oil 4% and DHA 2%**

| **INCI** | **Weight (%)** |
|---|---|
| Aqua | 45.0000 |
| Aqua, Hydroxypropyl Starch Phosphate | 2.5000 |
| Glycerin | 6.0000 |
| succinoglucam gum | 0.1500 |
| p-anisic acid | 0.1000 |
| Aqua, Sodium Hydroxide | 0.0570 |
| Glyceryl Caprylate | 1.0000 |
| Aqua, Sorbitol | 1.5000 |
| Glyceryl Stearate, PEG-100 Stearate | 1.7000 |
| Arachidyl alcohol, Arachidyl Glucoside, Behenyl Alcohol | 3.0000 |
| Cetyl Alcohol | 1.5000 |
| Ethylhexyl Stearate | 11.0000 |
| Persea gratissima (avocado) oil | 4.0000 |
| Aqua | 15.6470 |
| Tocopheryl Acetate | 0.1000 |
| Cyclopentasiloxane, Cyclohexasiloxane | 4.0000 |
| Perfume, Dipropylene Glycol | 0.4000 |
| Aqua, Lactic Acid, Propylene Glycol, Aloe Barbadensis Leaf Juice | 0.1000 |
| Aqua, Citric Acid | 0.0246 |
| Aqua | 0.2214 |
| Dihydroxyacetone | 2.0000 |
| Total | 100.0000 |

**Table 8: a comparative example of an emulsion with jojoba oil 4% and DHA 2%**

| **INCI** | **Weight (%)** |
|---|---|
| Aqua | 45.000 |
| Aqua, Hydroxypropyl Starch Phosphate | 2.500 |
| Glycerin | 6.000 |
| succinoglucam gum | 0.150 |
| p-anisic acid | 0.100 |
| Aqua, Sodium Hydroxide | 0.057 |
| Glyceryl Caprylate | 1.000 |
| Aqua, Sorbitol | 1.500 |
| Glyceryl Stearate, PEG-100 Stearate | 1.700 |
| Arachidyl alcohol, Arachidyl Glucoside, Behenyl Alcohol | 3.000 |
| Cetyl Alcohol | 1.500 |
| Ethylhexyl Stearate | 11.000 |
| Simmondsia Chinensis (jojoba oil) | 4.000 |
| Tocopheryl Acetate | 0.100 |
| Aqua | 15.633 |
| Cyclopentasiloxane, Cyclohexasiloxane | 4.000 |
| Perfume, Dipropylene Glycol | 0.400 |
| Aqua, Lactic Acid, Propylene Glycol, Aloe Barbadensis Leaf Juice | 0.100 |
| Aqua, Citric Acid | 0.026 |
| Aqua | 0.234 |
| Dihydroxyacetone | 2.000 |
| Total | 100.000 |

The jojoba oil (***Simmondsia Chinensis**)* is a vegetable oil which contains less than 40% by weight of C₁₈ unsaturated fatty acids. It has the following composition:

**Fatty Acid weight %**

| Saturated C16 | Mononsatu rated C16 | Saturated C18 | Monounsatu rated C18 | Diunsaturated C18 | Triunsaturated C18 | Monounsaturated C20 | Saturated C22 | Monounsaturate C22 |
|---|---|---|---|---|---|---|---|---|
| Palmitic acid | | Stearic acid | Oleic acid | Linoleic acid | Linolenic acid | eicosanoic acid | behenic acid | |
| <3 | <1 | | 5-15 | <1 | <1 | 65-80 | | 10-22 |

**Table 9: an emulsion according to the present invention with avocado oil 4% and DHA 1.2%**

| **INCI Name** | **%wt** |
|---|---|
| Water | 40.000 |
| Water; Hydroxypropyl Starch Phosphate | 2.500 |
| Glycerin | 6.000 |
| Succinoglycan | 0.150 |
| Sodium Hydroxide | 0.007 |
| Water | 0.016 |
| p-Anisic Acid | 0.100 |
| Glyceryl Caprylate | 1.000 |
| Sorbitol; Water | 1.500 |
| PEG-100 Stearate; Glyceryl Stearate | 1.700 |
| Arachidyl Alcohol; Behenyl Alcohol; Arachidyl Glucoside | 3.000 |
| Cetyl Alcohol; Stearyl Alcohol; Myristyl Alcohol | 1.500 |
| Isopropyl Palmitate | 2.000 |
| Ethylhexyl Stearate | 5.000 |
| Cyclohexasiloxane; Cyclopentasiloxane | 4.000 |
| Persea Gratissima (Avocado) Oil | 4.000 |
| Ascorbyl Palmitate; Lecithin; Tocopherol | 0.020 |
| Vanillin | 0.010 |
| Water | 25.897 |
| Perfume | 0.400 |
| Dihydroxyacetone | 1.200 |
| **Total** | 100.000 |

The process for preparing the emulsion of Table 9 was as follows:

In a main vessel, 40 % of total PURIFIED Water amount was added, and then Structure XL was added in cool water under stirring and homogenized until complete dispersion.

The composition was thus heated to 75°C. During the heating phase Glycerin and Rheozan were added and mixed until complete dissolution. The composition was neutralized with NaOH 30% and mixed for 15 minutes. Then Dermosoft 688 was added. Then Dermosoft GMCY and Neosorb 70/02 were added.

At 70°C, Simulsol 165, Montanov 202 and Cetyl Alcohol were added, then at 80°C Cetiol 868, Dow Coming 246, Isopropyl palmitate, Avocado oil were added. Emulsification took place during 25 minutes.

At 60°C Ronoxan, and Rhovanil Natural were added and mixed for 15mn then the remainder amount of water was added.

At 35°C, DHA powder was added under homogenizer and stirring for 20 minutes, then fragrance Glitter was added. The viscosity and pH were checked.

**Table 10: an emulsion according to the present invention borage oil 2% and DHA 1.2%**

| **INCI Name** | **%wt** |
|---|---|
| Water | 40.000 |
| Water; Hydroxypropyl Starch Phosphate | 2.500 |
| Glycerin | 6.000 |
| Succinoglycan | 0.150 |
| Sodium Hydroxide | 0.007 |
| Water | 0.016 |
| p-Anisic Acid | 0.100 |
| Glyceryl Caprylate | 1.000 |
| Sorbitol; Water | 1.500 |
| PEG-100 Stearate; Glyceryl Stearate | 1.700 |
| Arachidyl Alcohol; Behenyl Alcohol; Arachidyl Glucoside | 3.000 |
| Cetyl Alcohol; Stearyl Alcohol; Myristyl Alcohol | 1.500 |
| Isopropyl Palmitate | 2.000 |
| Ethylhexyl Stearate | 7.000 |
| Cyclohexasiloxane; Cyclopentasiloxane | 4.000 |
| Ascorbyl Palmitate; Lecithin; Tocopherol | 0.020 |
| Vanillin | 0.010 |
| Borago Officinalis (Borage) Seed Oil; Tocopherol | 2.000 |
| Water | 25.897 |
| Perfume | 0.400 |
| Dihydroxyacetone | 1.200 |
| Total | 100.000 |

The process for preparing the emulsion of Table 10 was as follows:

In a main vessel, 40 % of total PURIFIED Water amount was added, and then Structure XL was added in cool water under stirring and homogenized until complete dispersion.

The composition was thus heated to 75°C. During the heating phase Glycerin and Rheozan were added and mixed until complete dissolution. The composition was neutralized with NaOH 30% and mixed for 15 minutes. Then Dermosoft 688 was added. Then Dermosoft GMCY and Neosorb 70/02 were added.

At 70°C, Simulsol 165, Montanov 202 and Cetyl Alcohol were added, then at 80°C Cetiol 868, Dow Coming 246 and Isopropyl palmitate were added. Emulsification takes place during 25 minutes.

At 60°C Ronoxan, Akostar and Rhovanil Natural were added and mixed for 15mn then the remainder amount of water was added.

At 35°C, DHA powder was added under homogenizer and stirring for 20 minutes, then fragrance Glitter was added. The viscosity and pH were checked.

**Table 11: a comparative example of an emulsion with ethylhexyl stearate and DHA 2%**

| **INCI Name** | **%wt** |
|---|---|
| Perfume | 0.400 |
| Citric Acid | 0.040 |
| Cetyl Alcohol; Stearyl Alcohol; Myristyl Alcohol | 1.500 |
| Ethylhexyl Stearate | 11.000 |
| Tocopheryl Acetate (vitamin E) | 0.100 |
| Water | 64.992 |
| Glycerin | 6.000 |
| Dihydroxyacetone | 1.900 |
| Arachidyl Alcohol; Behenyl Alcohol; Arachidyl Glucoside | 3.000 |
| Sodium Hydroxide | 0.018 |
| PEG-100 Stearate; Glyceryl Stearate | 1.700 |
| Sorbitol; Water | 1.500 |
| Aloe Barbadensis Leaf Extract | 0.100 |
| Glyceryl Caprylate | 1.000 |
| Water; Hydroxypropyl Starch Phosphate | 2.500 |
| Cyclohexasiloxane; Cyclopentasiloxane | 4.000 |
| p-Anisic Acid | 0.100 |
| Succinoglycan | 0.150 |
| Total | 100.000 |

These emulsions were applied on the skin and the color intensity was evaluated according to the following protocol: Each of the emulsions were applied to six women ranging in age between 18 and 60 years old having phototype I and II.

The emulsions were applied to the forearms, to separate zones by light massage until product penetration.

The primary objective of the study was to evidence, on volunteers, the effect of the tested products on skin color, by a clinical evaluation.

### METHOD

This was a double-blind, randomized, intra-individual study. Each subject was her own control.

### ASSESSMENT CRITERIA

- Scoring of the color intensity was performed by a technician using analogical scales.
- The treated zones were classified from the more colored to the less colored.

### SCORING BY TECHNICIAN

The technician in charge of the study carried out a visual scoring of skin color after 5 daily applications and 3 hours after the last application, with the following visual analogical scale:
Intensity of skin pigmentation

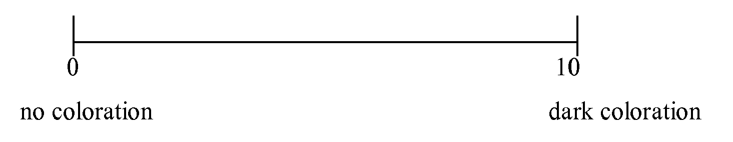

For each volunteer, the technician also classified the products from the more colored to the less colored after 5 daily applications.

### TRIAL SCHEDULE

### On Day 0

- The subjects came to the laboratory without having applied any product to their forearms since the previous evening.
- Six zones on the forearms of each subject were determined (one zone per tested product).
- The technician applied each product to the corresponding zone (1 µl/cm²).

### On Days I through 4

- The subjects evaluated the color visibility and intensity on each zone and classified the products from the more colored to the less colored.
- The technician applied each product to its corresponding zone (1 ul/cm²).

### On Day 4, three hours after last application

- Three hours after the last application on day 4, the subjects evaluated the color visibility and intensity on each zone and classified the products from the more colored to the less colored.
- The technician performed visual scoring of the coloration Intensity on each zone and classified the products from the more colored to the less colored.

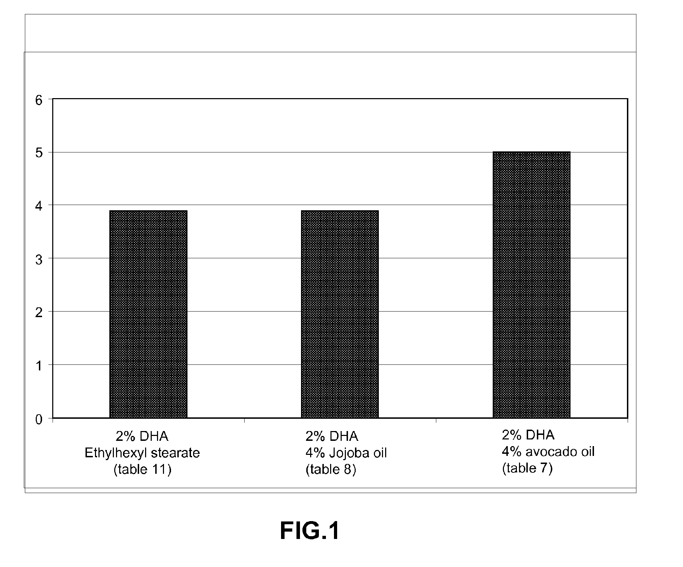

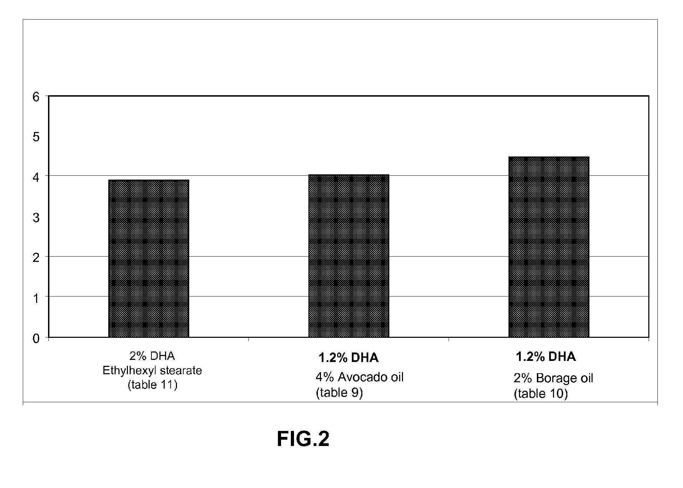

The results are indicated in Figures 1 and 2.

As can be seen from the results, the color intensity of the emulsion containing 2% DHA and avocado oil (Table 7) is higher than a comparative emulsion which does not contain avocado oil but jojoba oil (Table 8) or ethylhexyl stearate alone (Table 11) for the same amount of DHA (2% by weight).

The color intensity of the emulsion containing 1.2% DHA and avocado oil (Table 9) is equivalent to a comparative emulsion which does not contain avocado oil but ethylhexyl stearate alone (Table 11) for a higher amount of DHA (2% by weight). The color intensity of the emulsion containing 1.2% DHA and borage oil (Table 10) is higher than a comparative emulsion which does not contain borage oil but ethylhexyl stearate alone (Table 11) for the a higher amount of DHA (2% by weight).

Therefore, it is possible for the same type of emulsion, to decrease significantly (by 40%) the quantity of DHA by using borage oil or avocado oil in the emulsion.

## Claims

1. A sunless skin tanning oil-in-water emulsion comprising:
a) DHA, and
b) a vegetable oil comprising a C₁₈ unsaturated fatty acid fraction, wherein the C₁₈ unsaturated fatty acid fraction is present in a weight ratio of C₁₈ unsaturated fatty acid fraction to DHA that is at least about 1:2.

2. The oil-in-water emulsion according to claim 1, wherein it comprises less than 2 % by weight of DHA relative to the total weight of the emulsion, advantageously less than 1.5 % by weight of DHA relative to the total weight of the emulsion.

3. The oil-in-water emulsion according to claim 1 or 2, wherein the vegetable oil comprises a linoleic acid fraction, wherein the linoleic acid fraction is present in a weight ratio of linoleic acid fraction to DHA that is at least about 1:3, advantageously at least about 1:2, more advantageously at least about 1:1.5.

4. The oil-in-water emulsion according to any one of claims 1 to 3, wherein the vegetable oil contains more than 40 % of monounsaturated and diunsaturated C18 fatty acids.

5. The oil-in-water emulsion according to claim 4, wherein the vegetable oil is selected from the group consisting of avocado oil, borage oil, cottonseed oil, sesame oil and a mixture thereof, advantageously in the group consisting of avocado oil, borage oil and a mixture thereof.

6. The oil-in-water emulsion according to any one of claims 1 to 5, wherein it is free of color additive.

7. The oil-in-water emulsion according to any one of claims 1 to 6, wherein it is free of mineral oil and petrolatum.

8. The oil-in-water emulsion according to any one of claims 1 to 7, wherein it comprises a gelling agent selected from the group consisting of a modified starch gelling agent, a branched polysaccharide gelling agent and a mixture thereof.

9. A method of tanning skin comprising applying the oil-in-water emulsion of any one of claims 1 to 8 to the skin.

10. Use of a vegetable oil comprising a C₁₈ unsaturated fatty acid fraction to intensify the coloring action of DHA on the skin, wherein the C₁₈ unsaturated fatty acid fraction is present in a weight ratio of C₁₈ unsaturated fatty acid fraction to DHA that is at least about 1:2.

## Amended claims

### Amended claims in accordance with Rule 137(2) EPC.

**1.** A sunless skin tanning oil-in-water emulsion comprising:
a) DHA as the only tanning agent, and
b) a vegetable oil comprising a C₁₈ unsaturated fatty acid fraction, wherein the C₁₈ unsaturated fatty acid fraction is present in a weight ratio of C₁₈ unsaturated fatty acid fraction to DHA that is at least about 1:2.

**2.** The oil-in-water emulsion according to claim 1, wherein it comprises less than 2 % by weight of DHA relative to the total weight of the emulsion, advantageously less than 1.5 % by weight of DHA relative to the total weight of the emulsion.

**3.** The oil-in-water emulsion according to claim 1 or 2, wherein the vegetable oil comprises a linoleic acid fraction, wherein the linoleic acid fraction is present in a weight ratio of linoleic acid fraction to DHA that is at least about 1:3, advantageously at least about 1:2, more advantageously at least about 1:1.5.

**4.** The oil-in-water emulsion according to any one of claims 1 to 3, wherein the vegetable oil contains more than 40 % of monounsaturated and diunsaturated C18 fatty acids.

**5.** The oil-in-water emulsion according to claim 4, wherein the vegetable oil is selected from the group consisting of avocado oil, borage oil, cottonseed oil, sesame oil and a mixture thereof, advantageously in the group consisting of avocado oil, borage oil and a mixture thereof.

**6.** The oil-in-water emulsion according to any one of claims 1 to 5, wherein it is free of color additive.

**7.** The oil-in-water emulsion according to any one of claims 1 to 6, wherein it is free of mineral oil and petrolatum.

**8.** The oil-in-water emulsion according to any one of claims 1 to 7, wherein it comprises a gelling agent selected from the group consisting of a modified starch gelling agent, a branched polysaccharide gelling agent and a mixture thereof.

**9.** A method of tanning skin comprising applying the oil-in-water emulsion of any one of claims 1 to 8 to the skin.

**10.** Use of a vegetable oil comprising a C₁₈ unsaturated fatty acid fraction to intensify the coloring action of DHA on the skin, wherein the C₁₈ unsaturated fatty acid fraction is present in a weight ratio of C₁₈ unsaturated fatty acid fraction to DHA that is at least about 1:2.
